# EUROPEAN PATENT SPECIFICATION

(11) **EP 2 668 937 B1**
(45) Date of publication and mention of the grant of the patent: **29.06.2016**
(21) Application number: 12169759.3
(22) Date of filing: 29.05.2012
(51) Int. Cl.: A61F 15/00, A61F 5/448, A61M 27/00, A61M 1/00

(54) **Device for wound treatment**
Vorrichtung zur Wundbehandlung
Dispositif pour le traitement des plaies

(43) Date of publication of application: 04.12.2013
(73) Proprietor: Stapf, Michael, 65594 Runkel (DE)
(72) Inventor: Stapf, Michael, 65594 Runkel (DE)
(74) Representative: Mill, Andreas

(56) References cited:
- WO-A1-95/17146
- WO-A1-2007/045845
- US-A- 2 524 750
- US-A- 4 078 568
- US-A- 4 460 363
- US-A- 4 775 374
- US-A- 4 778 446
- US-A1- 2008 119 804

## Description

The present invention is directed to a device for wound treatment at a body part of a human being or an animal, in particular for open wound treatment.

Systems for wound treatment, in particular for open wound treatment, have been known for a long time. Today for classical wound treatment dressings in most cases a type of cloth is used which consists of several layers. A dressing is used for application to a wound to promote healing and/or prevent further harm. Such dressings for wound treatment are wrapped as a bandage around the affected body part. Alternatively, closed tubes can be used which are slipped over the affected body part. For dressings which are additionally necessary to hold the body part in place until healing is confirmed, rigid materials are used with the dressing, for example a splint made of metal i.e. in form of a wire ladder. In many cases the dressing material comprises a pharmaceutical product in the area of the wound which positively affects healing of the wound after application of the dressing.

For the treatment of an ileostoma today, often a base waver is used which is glued on the abdominal wall having a pouch attached thereto, which serves to collect the intestinal waste. For this, there are one or two piece systems on the market. The one piece system comprises the base waver which is rigidly connected to the pouch and the system can only be changed as a whole. The two piece system comprises the base waver and the pouch as separate units so that the waver can stay on the abdominal wall during change of the pouch because of hygienic reasons. Further, the pouch can be secured at the lower end with a leak-proof clip or velcro fastening so that the pouch can be emptied several times a day without changing the pouch. US-446036363 and US-4775374 disclose such systems.

A similar dressing system is disclosed in WO-9517146.

For a fast and uncomplicated healing of wounds without much pain, in particular of open wounds, today's systems are often insufficient. In particular during treatment with pharmaceutical products or for wound analysis, the cloth dressing of wound treatment systems has a lot of disadvantages.

It is therefore an object of the present invention to provide a device which offers new possibilities of wound treatment and wound analysis.

This is obtained by a device as defined in claim 1 or 2.

The main aspect of the invention is that the device comprises a stiff hollow body forming a chamber and a plate connected or connectable with the hollow body, wherein the plate forms a seat area for attachment of the device to the wounded body part. The hollow body and the plate each comprise a first through opening, wherein the first opening is situated at the distal end of the hollow body. The first openings of the hollow body and the plate enclose or surround the wound if the inventive device has been attached to the affected body part. At the proximal end of the hollow body the hollow body provides at least one second through opening, wherein the at least one second opening is preferably closable.

In an alternative of the present invention the inventive device comprises a stiff hollow body forming a chamber and a flange element, wherein the flange element is situated at the distal end of the hollow body. The flange element forms a seat area for attachment of the hollow body at the affected body part. The hollow body comprises at its distal end a first through opening and at its proximal end at least one second through opening, wherein the at least one second through opening is preferably closable. The first opening of the hollow body surrounds or encloses the wound if the inventive device has been attached to the affected body part. The hollow body and the flange element are preferably formed integrally.

Through the at least one second opening at the proximal end of the hollow body the wound can be treated or analyzed from outside.

The advantage of the inventive device consists therein that the chamber formed by the stiff hollow body which is accommodated above the wound and which is referred to as wound chamber in the following, allows simple and frequent change of dressing material or a wound treatment by irrigation using medicaments, for example using an anti-biotic, an enzyme or a nutrient solution. This cleansing treatment can be executed not only for a short time but with the inventive device over hours, i.e. by a continuous flow utilizing a pump. It is further of advantage that the wound chamber forms a closed system providing a reproducible possibility for extraction of exudate for wound area analysis. Further, by the first through opening and the wound chamber, the wound and the wound area, respectively are defined precisely so that, in particular during animal experiments, reproducible and scientifically standardized results can be achieved.

According to a preferred embodiment, the hollow body may comprise at its distal end means for sealing of wound in lateral direction, preferably a sealing lip. Preferably, such sealing means, for example the sealing lip, form a circumferential element at the rim of the first through opening. The sealing lip prevents leaking exudate or a medicament, for example wound irrigation liquid, leaking from the wound chamber laterally.

Such devices may be easily produced if the hollow body is formed as a hollow cylinder or a hollow prism having at least three sides, i.e. forming at least a triangular prism. The use of hollow four-, five- or six-sided prisms or prisms with more than six side surfaces is possible as well. The cross-sectional area of the hollow space of the hollow body may be circular or triangular or polygonal. Any other user-defined form of the hollow body may be used as well.

In order to close the at least one second through opening, the inventive device may comprise a lid or cover. The lid may be rigidly or removable connected to the device or may be formed as fully separate element. Preferably, the lid and the at least one second opening of the inventive device may form a screw closure or snap lock.

According to a preferred embodiment the plate or the flange element may comprise at least one stiffening rib, for example four stiffening ribs, so that the plate or the flange element may be formed thinner, more light weight and may therefore be produced more cost-efficient, though the plate or the flange element has sufficient stiffness.

The plate or the flange element may be preferably formed in a way that the respective distal side serves as a seat area for attachment of the device to the affected body part.

The seat area may be formed in a way that it may be placed beside the wound if the seat area has been attached to the affected body part so that the wound itself remains free and surrounded or enclosed by the first opening of the plate or the hollow body or the flange element. The wound chamber of the hollow body is accommodated above the wound which means it is situated in proximal direction of the wound. Hence, the wound may be treated and analyzed easily without additional injuries/impact of the wound by the inventive device. Preferably, the plate forming the seat area may be connected with the distal end of the hollow body.

The distal seat area of the plate or the flange element may comprise in a preferred embodiment at least partly a glue and/or a medicament, in particular a coating comprising a glue and/or a medicament. Alternatively, the glue and/or medicament may be contained within the plate or flange element and released during attachment at the affected body part. With the glue the device may be permanently attached to the affected body part, in particular to the skin of the affected body part. As a glue, preferably a bio-compatible glue, for example an organ glue (Octyl-Cyanoacrylate) True Glue, Gelatin-Resorcin-Formalin (GRF) glue, latex glue (SAUER skin glue) may be used. The specification of the glue may be varied according to the stage of the healing process of the wound and the medication so that an optimal endurance of the dressing is achieved. The medicament may enhance the body's compatibility for the plate or the flange element, in particular the skin's compatibility. For example the SAUER skin glue "Original" enhances the skin's compatibility for the plate or the flange element, because this glue comprises latex as glue, heptane as a solvent, zinc oxide as skin protection and lanolin for skin care.

The term "medicament", as used herein, means a pharmaceutical formulation containing at least one pharmaceutically active compound,
wherein in one embodiment the pharmaceutically active compound has a molecular weight up to 1500 Da and/or is a peptide, a protein, a polysaccharide, a vaccine, a DNA, a RNA, , an enzyme, an antibody or a fragment thereof, a hormone or an oligonucleotide, or a mixture of the above-mentioned pharmaceutically active compound, wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exendin-3 or exendin-4 or an analogue or derivative of exendin-3 or exendin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyhepta¬decanoyl) human insulin.

Exendin-4 for example means Exendin-4(1-39), a peptide of the sequence H His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2, H-(Lys)5-des Pro36, des Pro37 Exendin-4(1-39)-NH2, des Pro36 Exendin-4(1-39), des Pro36 [Asp28] Exendin-4(1-39), des Pro36 [IsoAsp28] Exendin-4(1-39), des Pro36 [Met(O)14, Asp28] Exendin-4(1-39), des Pro36 [Met(O)14, IsoAsp28] Exendin-4(1-39), des Pro36 [Trp(02)25, Asp28] Exendin-4(1-39), des Pro36 [Trp(02)25, IsoAsp28] Exendin-4(1-39), des Pro36 [Met(O)14 Trp(02)25, Asp28] Exendin-4(1-39), des Pro36 [Met(O)14 Trp(02)25, IsoAsp28] Exendin-4(1-39); or
des Pro36 [Asp28] Exendin-4(1-39), des Pro36 [IsoAsp28] Exendin-4(1-39), des Pro36 [Met(O)14, Asp28] Exendin-4(1-39), des Pro36 [Met(O)14, IsoAsp28] Exendin-4(1-39), des Pro36 [Trp(02)25, Asp28] Exendin-4(1-39), des Pro36 [Trp(02)25, IsoAsp28] Exendin-4(1-39), des Pro36 [Met(O)14 Trp(02)25, Asp28] Exendin-4(1-39), des Pro36 [Met(O)14 Trp(02)25, IsoAsp28] Exendin-4(1-39), wherein the group -Lys6-NH2 may be bound to the C-terminus of the Exendin-4 derivative;
or an Exendin-4 derivative of the sequence des Pro36 Exendin-4(1-39)-Lys6-NH2 (AVE0010), H-(Lys)6-des Pro36 [Asp28] Exendin-4(1-39)-Lys6-NH2, des Asp28 Pro36, Pro37, Pro38Exendin-4(1-39)-NH2, H-(Lys)6-des Pro36, Pro38 [Asp28] Exendin-4(1-39)-NH2, H-Asn-(Glu)5des Pro36, Pro37, Pro38 [Asp28] Exendin-4(1-39)-NH2, des Pro36, Pro37, Pro38 [Asp28] Exendin-4(1-39)-(Lys)6-NH2, H-(Lys)6-des Pro36, Pro37, Pro38 [Asp28] Exendin-4(1-39)-(Lys)6-NH2, H-Asn-(Glu)5-des Pro36, Pro37, Pro38 [Asp28] Exendin-4(1-39)-(Lys)6-NH2, H-(Lys)6-des Pro36 [Trp(O2)25, Asp28] Exendin-4(1-39)-Lys6-NH2, H-des Asp28 Pro36, Pro37, Pro38 [Trp(02)25] Exendin-4(1-39)-NH2, H-(Lys)6-des Pro36, Pro37, Pro38 [Trp(02)25, Asp28] Exendin-4(1-39)-NH2, H-Asn-(Glu)5-des Pro36, Pro37, Pro38 [Trp(02)25, Asp28] Exendin-4(1-39)-NH2, des Pro36, Pro37, Pro38 [Trp(02)25, Asp28] Exendin-4(1-39)-(Lys)6-NH2, H-(Lys)6-des Pro36, Pro37, Pro38 [Trp(02)25, Asp28] Exendin-4(1-39)-(Lys)6-NH2, H-Asn-(Glu)5-des Pro36, Pro37, Pro38 [Trp(02)25, Asp28] Exendin-4(1-39)-(Lys)6-NH2, H-(Lys)6-des Pro36 [Met(O)14, Asp28] Exendin-4(1-39)-Lys6-NH2, des Met(O)14 Asp28 Pro36, Pro37, Pro38 Exendin-4(1-39)-NH2, H-(Lys)6-desPro36, Pro37, Pro38 [Met(O)14, Asp28] Exendin-4(1-39)-NH2, H-Asn-(Glu)5-des Pro36, Pro37, Pro38 [Met(O)14, Asp28] Exendin-4(1-39)-NH2, des Pro36, Pro37, Pro38 [Met(O)14, Asp28] Exendin-4(1-39)-(Lys)6-NH2, H-(Lys)6-des Pro36, Pro37, Pro38 [Met(O)14, Asp28] Exendin-4(1-39)-(Lys)6-NH2, H-Asn-(Glu)5 des Pro36, Pro37, Pro38 [Met(O)14, Asp28] Exendin-4(1-39)-(Lys)6-NH2, H-Lys6-des Pro36 [Met(O)14, Trp(02)25, Asp28] Exendin-4(1-39)-Lys6-NH2, H-des Asp28 Pro36, Pro37, Pro38 [Met(O)14, Trp(02)25] Exendin-4(1-39)-NH2, H-(Lys)6-des Pro36, Pro37, Pro38 [Met(O)14, Asp28] Exendin-4(1-39)-NH2, H-Asn-(Glu)5-des Pro36, Pro37, Pro38 [Met(O)14, Trp(02)25, Asp28] Exendin-4(1-39)-NH2,
   des Pro36, Pro37, Pro38 [Met(O)14, Trp(02)25, Asp28] Exendin-4(1-39)-(Lys)6-NH2, H-(Lys)6-des Pro36, Pro37, Pro38 [Met(O)14, Trp(02)25, Asp28] Exendin-4(S1-39)-(Lys)6-NH2,
   H-Asn-(Glu)5-des Pro36, Pro37, Pro38 [Met(O)14, Trp(02)25, Asp28] Exendin-4(1-39)-(Lys)6-NH2;
or a pharmaceutically acceptable salt or solvate of any one of the afore-mentioned Exendin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies are globular plasma proteins (~150 kDa) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two β sheets create a "sandwich" shape, held together by interactions between conserved cysteines and other charged amino acids.

There are five types of mammalian Ig heavy chain denoted by α, δ, ε, γ, and µ. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (CH) and the variable region (VH). In one species, the constant region is essentially identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals, there are two types of immunoglobulin light chain denoted by A and κ. A. light chain has two successive domains: one constant domain (CL) and one variable domain (VL). The approximate length of a light chain is 211 to 217 amino acids. Each antibody contains two light chains that are always identical; only one type of light chain, κ or λ, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1 C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

According to the invention at least one, preferably circumferential, rip is provided at the inner surface of the hollow body. This rib fixes the material which is inserted into the wound chamber above the wound for wound treatment. Therefore, the rib provides a projection or nose. The material, for example a pad soaked with a medicament, is prevented from detachment by rib.

In a further preferred embodiment the plate or the flange element may comprise means for operating the device, for example a hook or a recess. The means for operating allow the device to be easily sterilized or attached to the treated body part in a sterile manner. The hook for example simplifies hanging the device up in a sterilizing oven.

Further, it is advantageous if the at least one second opening comprises a coupling and/or connecting element. By the coupling and/or connecting element, for example, by an element of the bayonet joint or a screw, it allows to connect the device preferably leak-proof with a closure, a tube, an extraction device or the like in order to treat, protect or analyze the wound. For example anti-biotic, enzyme or nutrient solutions may be inserted into or extracted from the wound chamber by a tube which may be connected to a pump. The coupling and/or connecting element allows to connect the device with a coupling and/or connecting element of an extraction device, for example a syringe, in order to extract exudate from the wound e.g. for analysis which is particularly advantageous for wound treatment experiments using animals. In this case the inventive device comprises two or more second through openings, which may be accommodated at the proximal end of the hollow body at an opposite site of the wound or within the outer surface of the hollow body.

The height of the hollow body may be approximately between 5 mm and 50 mm, in particular between 5 mm and 30 mm.

The person skilled in the art understands that the present invention is not restricted to the explained possibilities.

The above-mentioned advantages as well as other advantages of various aspects of the present invention will become apparent to those of ordinary skill in the art by reading the following detailed description with the explanations of the accompanying drawings.

Exemplary embodiments of the present invention are described herein with reference to schematic drawings in which:

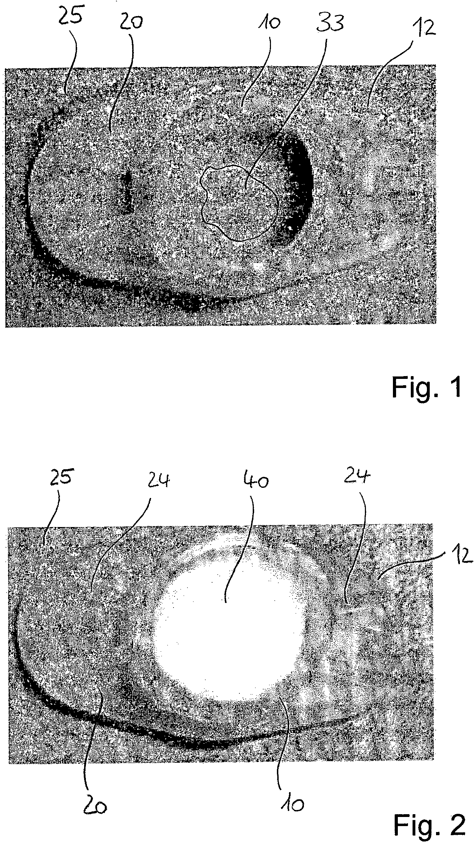

Fig. 1 illustrates a perspective view of an embodiment of the inventive device from above attached to an affected body part,
Fig. 2 the embodiment of Fig. 1 in a view according to Fig. 1 containing a dressing cloth inlet,
Fig. 3 the embodiment of Fig. 1 in a view according to Fig. 1 with a lid and
Fig. 4 the embodiment of Fig. 1 in a longitudinal cross section.

The embodiment of the inventive device depicted in the figures comprises a hollow body 10 in form of a hollow cylinder and a flange element 20 (hereinafter short flange). The flange 20 has an outer contour with a rhombohedral form. The flange 20 is connected to the distal end 11 of the hollow body 10 and is formed integrally with it. The flange 20 has rounded corners and a circumferential bulge 12 at its boundary.

The hollow body 10 comprises at its distal end 11 one basically circular first through opening 13 enclosing the wound 33 if the device has been attached to the affected body part. Hence, there are different inventive devices which have respective, different diameters of the first opening 13 for different wound sizes.

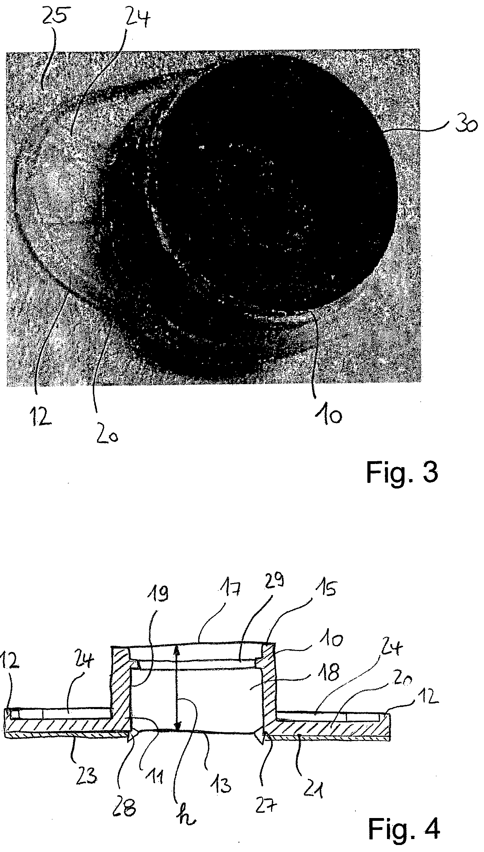

The hollow body 10 comprises at its opposing, proximal end 15 a second through opening 17 which is closable by a lid 13 or cover. The lid is depicted in Fig. 3. The second opening 17 may be used in order treat the wound 33 or analyze it and to make the wound accessible.

The hollow body 10 forms with its inner volume a wound chamber 18 sitting above the wound 33 if the device has been attached to the affected body part of the human being or the animal, in particular to the skin 25 of the affected body part.

The surface of the inventive device at the distal side of flange 20 serves as a seat area 21 for application of the inventive device to the affected body part with the wound 33 and for attachment at the affected body part, wherein the first opening 13 is chosen in a size that the wound 33 is fully contained within the rim of the first opening 13 and that the seat area 21 does not touch the wound 33 but is only placed at the skin 25 laterally to the wound 33.

In a preferred embodiment the seat area 21 may comprise at least partly a layer with a bio-compatible glue in order to firmly attach the device to the particularly affected body part.

On the proximal side the flange 20 comprises four stiffening ribs 24, which run approximately radially to the longitudinal axis of the hollow body 10. These stiffening ribs 24 enhance the stiffness of the flange 20 and minimize the material consumption. A circumferential sealing lip 28 is accommodates at the rim 27 of the first opening 13 of the hollow body 10 which seals the wound area and the wound chamber 18, respectively in lateral direction. The sealing lip 28 is made from a flexible material which tightly sits on the affected body part when the inventive device has been placed at this body part.

At its inner surface 19 the hollow body 10 comprises a circumferential rip 29 in order to fix the material inserted into the wound chamber 18 for the treatment of the wound 33, for example a dressing cloth insert 14 (see fig: 2). Hence, the insert cannot be easily detached from the wound chamber 18, but will be retained by the projection which is formed by rib 29. If necessary, a plurality of such ribs or similar attachment means may be provided.

The height h of the hollow body 10 and the wound chamber 18, respectively is preferably between 5 mm and 50 mm, particularly preferably between 5 mm and 30 mm.

As a material for the inventive device, that means for the hollow body 10, the flange 20 or the plate and the lid, preferably plastics which are approved for medical products may be used, for example polypropylene, polyethylene terephthalate, latex etc.

With the inventive device a new possibility is provided to treat wounds 33 at the body surface of a human being or an animal easily and reproducible, in particular open wounds. Because the wound area is precisely defined, a scientific evaluation and standardization of the wound treatment experiment results using animals is possible. Additionally, from the invention arise new possibilities for wound analysis because the wound 33 is accessible at any time.

### List of reference signs

- 10: hollow body
- 11: distal end of the hollow body 10
- 12: bulge at the outer rim of the flange 20
- 13: first opening in hollow body 10
- 15: proximal end of hollow body 10
- 17: second opening in hollow body 10
- 18: wound chamber
- 19: inner surface of hollow body 10
- 20: flange or plate
- 21: seat area
- 23: glue coating
- 24: rib
- 25: skin
- 27: rim of first opening 13
- 28: sealing lip
- 29: rib
- 30: lid
- 33: wound
- 40: dressing cloth inlet
- h: height of hollow body 10

## Claims

1. Dressing for wound treatment at a wounded body part, in particular for open wound treatment, comprising a stiff hollow body (10) forming a chamber (18) and a plate connected or connectable with the hollow body (10), wherein said plate forms a seat area (21) for attachment of the dressing to said wounded body part, wherein said hollow body (10) and said plate each comprise a first through opening (13), wherein said first opening (13) is situated at the distal end (11) of said hollow body (10), wherein said hollow body (10) comprises at least one second through opening (17) at its proximal end (15), wherein said at least one second opening (17) is preferably closable, wherein at the inner surface (19) of said hollow body (10) there is at least one rib (29) which provides a projection or nose in order to fix a dressing material inserted into the wound chamber (18) for wound treatment so that said dressing material is prevented from detachment of the wound chamber (18).

2. Dressing for wound treatment at a wounded body part, in particular for open wound treatment, comprising a stiff hollow body (10) forming a chamber (18) and a flange element (20), wherein said flange element (20) is situated at the distal end (11) of the hollow body (10) and forms a seat area (21) for attachment of the hollow body (10) at said wounded body part, wherein said hollow body (10) comprises a first through opening (13) at its distal end (11) and at least one second through opening (17) at its proximal end (15), wherein said at least one second opening (17) is preferably closable, wherein at the inner surface (19) of said hollow body (10) there is at least one rib (29) which provides a projection or nose in order to fix a dressing material inserted into the wound chamber (18) for wound treatment so that said dressing material is prevented from detachment of the wound chamber (18).

3. Dressing according to claim 1 or 2, wherein said hollow body (10) comprises at its distal end (11) means for sealing the wound in lateral direction, wherein said means are preferably formed as a sealing lid (28).

4. Dressing according to any of the preceding claims, wherein said hollow body (10) is formed as a hollow cylinder or a hollow prism having at least three sides.

5. Dressing according to any of the preceding claims, wherein the dressing comprises a lid (30), wherein the lid (30) is used as a screw closure or snap lock for closure of said at least one second opening (17).

6. Dressing according to any of the preceding claims, wherein said plate forming the seat area (21) is connected with said distal end (11) of said hollow body (10).

7. Dressing according to any of the preceding claims, wherein said plate or said flange element (20) comprises at least one stiffening rib (24).

8. Dressing according to any of the preceding claims, wherein said seat area (21) of said plate or said flange element (20) has at least partly a glue (23) and/or a medicament.

9. Dressing according to any of the preceding claims, wherein the rib (29) at the inner surface (19) of said hollow body (10) is circumferential.

10. Dressing according to any of the preceding claims, wherein said plate or said flange element (20) each comprises means for operating the dressing, for example a hook.

11. Dressing according to any of the preceding claims, wherein said at least one second opening (17) comprises a coupling element and/or a connecting element.

12. Dressing according to claim 1 or 2, wherein the material for treatment of the wound is inserted into the wound chamber (18).

13. Dressing according to any of the claims 1 to 3, wherein the material for treatment of the wound is a dressing cloth insert (14).

14. Dressing according to any of the claims 1 to 3, wherein the material for treatment of the wound is a pad soaked with a medicament.

## Patentansprüche

1. Verband zur Wundbehandlung an einem verletzten Körperteil, insbesondere zur Behandlung offener Wunden, umfassend einen steifen Hohlkörper (10), der eine Kammer (18) bildet, und eine mit dem Hohlkörper (10) verbundene oder verbindbare Platte, wobei die Platte eine Sitzfläche (21) zum Anbringen des Verbands an dem verletzten Körperteil bildet, wobei der Hohlkörper (10) und die Platte jeweils eine erste Durchgangsöffnung (13) umfassen, wobei sich die erste Öffnung (13) am distalen Ende (11) des Hohlkörpers (10) befindet, wobei der Hohlkörper (10) mindestens eine zweite Durchgangsöffnung (17) an seinem proximalen Ende (15) umfasst, wobei die mindestens eine zweite Öffnung (17) vorzugsweise verschließbar ist, wobei es an der Innenfläche (19) des Hohlkörpers (10) mindestens eine Rippe (29) gibt, die einen Vorsprung oder eine Nase vorsieht, um ein in die Wundkammer (18) zur Wundbehandlung gelegtes Verbandsmaterial zu fixieren, sodass das Verbandsmaterial am Lösen aus der Wundkammer (18) gehindert ist.

2. Verband zur Wundbehandlung an einem verletzten Körperteil, insbesondere zur Behandlung offener Wunden, umfassend einen steifen Hohlkörper (10), der eine Kammer (18) und ein Flanschelement (20) bildet, wobei sich das Flanschelement (20) am distalen Ende (11) des Hohlkörpers (10) befindet und eine Sitzfläche (21) zum Anbringen des Hohlkörpers (10) an dem verletzten Körperteil bildet, wobei der Hohlkörper (10) eine erste Durchgangsöffnung (13) an seinem distalen Ende (11) und mindestens eine zweite Durchgangsöffnung (17) an seinem proximalen Ende (15) umfasst, wobei die mindestens eine zweite Öffnung (17) vorzugsweise verschließbar ist, wobei es an der Innenfläche (19) des Hohlkörpers (10) mindestens eine Rippe (29) gibt, die einen Vorsprung oder eine Nase vorsieht, um ein in die Wundkammer (18) zur Wundbehandlung gelegtes Verbandsmaterial zu fixieren, sodass das Verbandsmaterial am Lösen aus der Wundkammer (18) gehindert ist.

3. verbund nach Anspruch 1 oder 2, wobei der Hohlkörper (10) au seinem distalen Ende (11) eine Einrichtung zum Abdichten der Wunde in seitlicher Richtung umfasst, wobei die Einrichtung vorzugsweise als ein Dichtungsdeckel (28) ausgebildet ist.

4. Verband nach beliebigen der vorangehenden Absprüche, wobei der Hohlkörper (10) als ein Hohlzylinder oder ein Hohlprisma mit mindestens drei Seiten ausgebindet ist.

5. verband nach beliebigen der vorangehenden Ansprüche, wobei der Verband einen Deckel (30) umfasst, wobei der Deckel (30) als ein Schraubverschluss oder eine Schnappverriegelung zum Verschließen der mindestens einen zweiten Öffnung (17) verwendet ist.

6. Verband nach beliebigen der vorangehenden Ansprüche, wobei die Platte, die die Sitzfläche (21) bildet, mit dem distalen Ende (11) des Hohlkörpers (10) verbunden ist.

7. Verband nach beliebigen der vorangehenden Anspruche, wobei die Platte oder das Flanschelement (20) mindestens eine Versteifungsrippe (24) umfasst.

8. verband nach beliebigen der vorangehenden Anspräche, wobei die Sitzfläche (21) der Platte oder des Flanschelements (20) mindestens teilsweise einen Klebstoff (23) und/oder ein Medikament aufweist.

9. Verband nach beliebigen der vorangehenden Anspruche, wobei die Rippe (29) an der Innenfläche (19) des Hohlkörpers (10) umlaufend ist.

10. Verband nach beliebigen der vorangehenden Absprüche, wobei die Platte oder das Flanschelement (20) jeweils eine Einrichtung zum Betätigen des Verbands umfasst, beilspielsweise einen Haken.

11. Verband nach beliebigen der vorangehenden. Anspruche, wobei die mindestens eine zweite Öffnung (17) ein Koppelelement und/oder ein Verbindungselement umfasst.

12. Verband nach Anspruch 1 oder 2, wobei das Material zur Behandlung der Wunde in die Wundkammer (18) gesetzt wird.

13. Verband nach beliebigen der Ansprüche 1 bis 3, wobei das Material zur Behandlung der Wunde eine Verbandsstoffeinlage (14) ist .

14. Verband nach beliebigen der Absprüche 1 bis 3, wobei das Material zur Behandlung der Wunde ein mit einem Medikament getränkter Tampon ist.

## Revendications

1. Pansement pour le traitement d'une plaie sur une partie blessée du corps, notamment pour le traitement d'une blessure ouverte, comprenant un corps creux (10) rigide formant une chambre (18) et une plaque reliée ou pouvant être reliée au corps creux (10), ladite plaque formant une zone d'appui (21) pour la fixation du pansement à ladite partie blessée du corps, ledit corps creux (10) et ladite plaque comprenant chacun une première ouverture (13) traversante, ladite première ouverture (13) se trouvant à l'extrémité distale (11) dudit corps creux (10), ledit corps creux (10) comprenant au moins une deuxième ouverture (17) traversante à son extrémité proximale (15), ladite au moins une deuxième ouverture (17) pouvant de préférence être fermée, au moins une nervure de renforcement (29) se trouvant sur la surface intérieure (19) dudit corps creux (10), laquelle produit une projection ou un tenon servant à immobiliser un matériel de pansement inséré dans la chambre à blessure (18) pour le traitement de la blessure, de manière à empêcher ledit matériel du pansement de se détacher de la chambre à blessure (18).

2. Pansement pour le traitement d'une plaie sur une partie blessée du corps, notamment pour le traitement d'une blessure ouverte, comprenant un corps creux (10) rigide formant une chambre (18) et un élément formant bride (20), ledit élément formant bride (20) se trouvant à l'extrémité distale (11) du corps creux (10) et formant une zone d'appui (21) pour la fixation du corps creux à ladite partie blessée du corps, ledit corps creux (10) comprenant une première ouverture (13) traversante à son extrémité distale (11) et au moins une deuxième ouverture (17) traversante à son extrémité proximale (15), ladite au moins une deuxième ouverture (17) pouvant de préférence être fermée, au moins une nervure de renforcement (29) se trouvant sur la surface intérieure (19) dudit corps creux (10), laquelle produit une projection ou un tenon servant à immobiliser un matériel de pansement inséré dans la chambre à blessure (18) pour le traitement de la blessure, de manière à empêcher ledit matériel du pansement de se détacher de la chambre à blessure (18).

3. Pansement selon la revendication 1 ou 2, avec lequel ledit corps creux (10) comprend à son extrémité distale (11) des moyens pour rendre la blessure étanche dans le sens lateral, lesdits moyens étant de préférence réalisés sous la forme d'un couvercle d'étanchéité (28).

4. Pansement selon l'une quelconque des revendications précédentes, avec lequel ledit corps creux (10) est réalisé sous la forme d'un cylindre creux ou d'un prisme creux comportant au moins trois côtés.

5. Pansement selon l'une quelconque des revendications précédentes, avec lequel le pansement comprend un couvercle (30), le couvercle (30) étant utilisé comme fermoir à visser ou verrou à enclencher pour la fermeture de ladite au moins une deuxième ouverture (17).

6. Pansement selon l'une quelconque des revendications précédentes, avec lequel ladite plaque formant la zone d'assise (21) est reliée à ladite extrémité distale (11) dudit corps creux (10).

7. Pansement selon l'une quelconque des revendications précédentes, avec lequel ladite plaque ou ledit élément formant bride (20) comprend au moins une nervure de raidissement (24).

8. Pansement selon l'une quelconque des revendications précédentes, avec lequel ladite zone d'assise (21) de ladite plaque ou dudit élément formant bride (20) comporte au moins partiellement un adhésif (23) et/ou un médicament.

9. Pansement selon l'une quelconque des revendications précédentes, avec lequel la nervure de renforcement (29) sur la surface intérieure (19) dudit corps creux (10) est circonférentielle.

10. Pansement selon l'une quelconque des revendications précédentes, avec lequel ladite plaque ou ledit élément formant bride (20) comprend chacun des moyens pour manipuler le pansement, par exemple un crochet.

11. Pansement selon l'une quelconque des revendications précédentes, avec lequel ladite au moins une deuxième ouverture (17) comprend un élément de couplage et/ou un élément de connexion.

12. Pansement selon la revendication. 1 ou 2, avec lequel le matériel destiné au traitement de la blessure est inséré dans la chambre à blessure (18).

13. Pansement selon l'une quelconque des revendications 1 à 3, avec lequel le matériel destiné au traitement de la blessure est un insert en étoffe de pansement (14).

14. Passement selon l'une quelconque des revendications 1 à 3, avec lequel la matériel destiné au traitement de la blessure est une compresse imprégnée d'un médicament.
